# EUROPEAN PATENT APPLICATION

(11) **EP 4 141 801 A1**
(43) Date of publication of application: **01.03.2023**
(21) Application number: 21200691.0
(22) Date of filing: 04.10.2021
(51) Int. Cl.: G06T 19/20, G06F 3/048, G06T 7/10

(54) **INTERACTIVE HANDLING OF 3D IMAGES**

(30) Priority: 31.08.2021 US 202163238819 P
(71) Applicant: Koninklijke Philips N.V., 5656 AG Eindhoven (NL)
(72) Inventor: MORY, Benoit Jean-Dominique Bertrand Maurice, Eindhoven (NL); PANSE, Ashish Sattyavrat, Eindhoven (NL); KEENAN, Philip Joseph Jr., Eindhoven (NL); SALEHI, Leili, Eindhoven (NL); WEBB, Penelope Eugenia, Eindhoven (NL)
(74) Representative: Philips Intellectual Property & Standards

(57) **Abstract**

The present invention relates to interactive image processing. In order to provide an improved and facilitated interactive handling of 3D images, a device (10) is provided that has a data input (12), a data processor (14) and an output interface (16). The data input provides 3D data (18) representing a region of interest of an object and also provides a 3D user input (20) relating to a 3D presentation. The data processor renders a 3D presentation of a segmentation, which segmentation is based on the 3D data, for 3D displaying the 3D presentation to a user. The data processor transforms the 3D user input into 3D object data and adapts the 3D segmentation based on the transformed 3D object data. The data processor further renders an updated 3D presentation data (22) of the adapted segmentation for a modified 3D display to a user. The output interface is configured to provide the updated 3D presentation data to a user, for example via a display (26).

## Description

### FIELD OF THE INVENTION

The present invention relates to interactive handling of 3D images and relates in particular to a device for interactive segmentation of 3D images, to a system for interactive segmentation of 3D images and to a method for interactive segmentation of 3D images.

### BACKGROUND OF THE INVENTION

In image guided therapy, the user, e.g. a surgeon, is provided with images relating to the region of interest of a subject, for example, for guiding and also for information purposes. One aspect is to provide image information to improve the user's 3D interaction, such as in a cathlab. As an example, subject information is provided as segmentation. Due to workflow reasons, user interaction with segmentations is provided, such as annotating images. Another form of interaction relates to providing user feedback for identifying objects and manipulating segmentations, for example by using touch screens to handle 3D images. However, it has been shown that providing interaction in such manner may be cumbersome for the user and may also hamper other workflow activities.

### SUMMARY OF THE INVENTION

There may thus be a need to provide an improved and facilitated interactive handling of 3D images.

The object of the present invention is solved by the subject-matter of the independent claims; further embodiments are incorporated in the dependent claims. It should be noted that the following described aspects of the invention apply also for the device for interactive segmentation of 3D images, for the system for interactive segmentation of 3D images and for the method for interactive segmentation of 3D images.

According to the present invention, a device for interactive segmentation of 3D images is provided. The device comprises a data input, a data processor and an output interface. The data input is configured to provide 3D data representing a region of interest of an object. The data input is also configured to provide a 3D user input relating to a 3D presentation. The data processor is configured to render a 3D presentation of a segmentation, which segmentation is based on the 3D data, for 3D displaying the 3D presentation to a user. The data processor is also configured to transform the 3D user input into 3D object data. The data processor is further configured to adapt the 3D segmentation based on the transformed 3D object data. The data processor is furthermore configured to render an updated 3D presentation data of the adapted segmentation for a modified 3D display to a user. The output interface is configured to provide the updated 3D presentation data to a user.

As an effect, 3D rendering, i.e. stereoscopic rendering combines the continuously updated segmentation result and the input volume. The immediate stereoscopic visual feedback of the impact of 3D user inputs and image features on the segmentation result creates an intuitive segmentation experience with natural hand-eye coordination.

Thus, a real-time interactive segmentation workflow is provided that facilitates e.g. to delineate and to measure 3D structures of interest in 3D volumetric acquisitions with augmented or virtual reality devices.

The user, e.g. a radiologist or surgeon, is put in control by asking for the 3D input to guide and adapt the segmentation and annotation process.

According to an example, for the 3D segmentation, in a first option it is provided that the data input is configured to provide the 3D data representing the region of interest of the object, and the data processor is configured to generate the segmentation of the region of interest based on the 3D data.

According to an example, for the 3D segmentation, in a second option, the data input is configured to provide the 3D data representing the region of interest of the object as the 3D segmentation. As an option, in addition or alternatively, the data input is configured to provide a user interactive segmentation input.

According to an example, the input controller is configured to provide the user with an option of creating a mesh based on the user's interaction by the user's fingers and/or eye gaze. The input controller is also configured to provide the user with a further option of moving the mesh along the segmentation.

According to an example, the data processor is configured to provide interactive deformable 3D shape templates. Further, the data processor is configured to adapt the deformable shape templates based on at least one of the group of an interactive 3D user input and an optimized matching with the segmented 3D anatomical structure. In an option, in addition or alternatively, the deformable 3D shape templates comprise models representing implantable devices. The data processor is configured to adapt the models; and the output interface is configured to provide the 3D data of the models for manufacturing a subject-adapted implantable device.

According to an example, the input controller is configured to provide a pre-determined geometric form; and the data processor is configured to morph the geometric form to the segmentation. In an option, in addition or alternatively, the form comprises at least one of the group of:
- a line, such that an interactive line is generated that can be manipulated in the 3D image space by 3D user interaction;
- a circle, such that an interactive circle is generated that can be manipulated in the 3D image space by 3D user interaction; and
- a sphere, such that an interactive sphere is generated that can be manipulated in the 3D image space by 3D user interaction.

According to the present invention, also a system for interactive segmentation of 3D images is provided. The system comprises a device for interactive segmentation of 3D images according to one of the preceding examples. The system also comprises at least one 3D interactive user input device and at least one 3D presentation device. The 3D interactive user input device is configured to provide the 3D user input. The 3D presentation device is configured to provide the updated 3D presentation data as the modified 3D display to the user.

According to an example, the 3D interactive user input device comprises at least one of the group of a hand tracking device and an eye gaze tracking device. In an option, in addition or alternatively, the 3D presentation device is a stereoscopic display.

According to an example, the 3D interactive user input device and the 3D presentation device are provided as a head-mounted gear configured to be worn by the user. In an option, in addition or alternatively, for providing the 3D user input, the 3D presentation of the segmentation is configured to be registered with a 3D user input device. In an option, in addition or alternatively, the device for interactive segmentation of 3D images is provided remotely from the head-mounted gear.

According to an example, the 3D interactive user input device is configured to provide the 3D user input as segmentation constraints as correction instructions to modify the segmentation. The data processor is configured to revise the 3D segmentation representing the region of interest of the object based on the segmentation constraints. Further, the 3D presentation device is configured to provide the revised 3D presentation data as the modified 3D display to the user.

According to the present invention, also a method for interactive segmentation of 3D images is provided. The method comprises the following steps:
- providing a 3D data representing a region of interest of an object;
- rendering a 3D presentation of the segmentation, which segmentation is based on the 3D data, for 3D displaying the 3D presentation to a user;
- providing a 3D user input relating to the 3D presentation;
- transforming the 3D user input into 3D object data;
- adapting the 3D segmentation based on the transformed 3D object data;
- rendering updated 3D presentation data of the adapted segmentation for a modified 3D display to a user; and
- providing the updated 3D presentation data to a user.

According to an aspect, a user is provided with a 3D segmentation and the option of 3D interaction. One way of providing such 3D interaction is by spatial tracking of movements of the user's hands in a 3D space. Another way of providing such 3D interaction is by tracking the user's view and the respective focused points in space, i.e. by gaze tracking. The user can thus act in space and can, for example, virtually grab a presentation that is then adapted, i.e. manipulated according to the user's interaction. The presentation of the segmentation is provided in 3D. The interaction and its tracking is also provided in 3D. Further, also the presentation of the feedback, i.e. the adapted segmentation is provided as a real 3D presentation.

This allows an intuitive user experience and provides improvements in terms of information to the user and feedback in real-time in a spatial context. This is in particular useful when dealing with complex anatomical structures. The 3D interaction provides a facilitated and thus faster and more efficient image processing where user interaction is required.

According to an aspect, a device is provided that has a data input, a data processor and an output interface. The data input provides 3D data representing a region of interest of an object and also provides a 3D user input relating to a 3D presentation. The data processor renders a 3D presentation of a segmentation, which segmentation is based on the 3D data, for 3D displaying the 3D presentation to a user. The data processor transforms the 3D user input into 3D object data and adapts the 3D segmentation based on the transformed 3D object data. The data processor further renders an updated 3D presentation data of the adapted segmentation for a modified 3D display to a user. The output interface provides the updated 3D presentation data to a user, for example via a display.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for interactive segmentation of 3D images.
Fig. 2 schematically shows a system for interactive segmentation of 3D images comprising the device of Fig. 1.
Fig. 3 shows steps of an example of a method for interactive segmentation of 3D images.
Fig. 4 shows a functional setup of another example of a system for interactive segmentation of 3D images.
Fig. 5 shows a functional setup of a further example of a system for interactive segmentation of 3D images.
Fig. 6 shows an example of user 3D interaction in 3D image.
Fig. 7 shows another example of user 3D interaction.
Fig. 8 shows a further example of user 3D interaction.
Fig. 9 shows a still further example of a user 3D interaction.
Fig. 10 shows further examples of a user 3D interaction.
Fig. 11 shows a still further example of a user 3D interaction in the context of an operation room.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

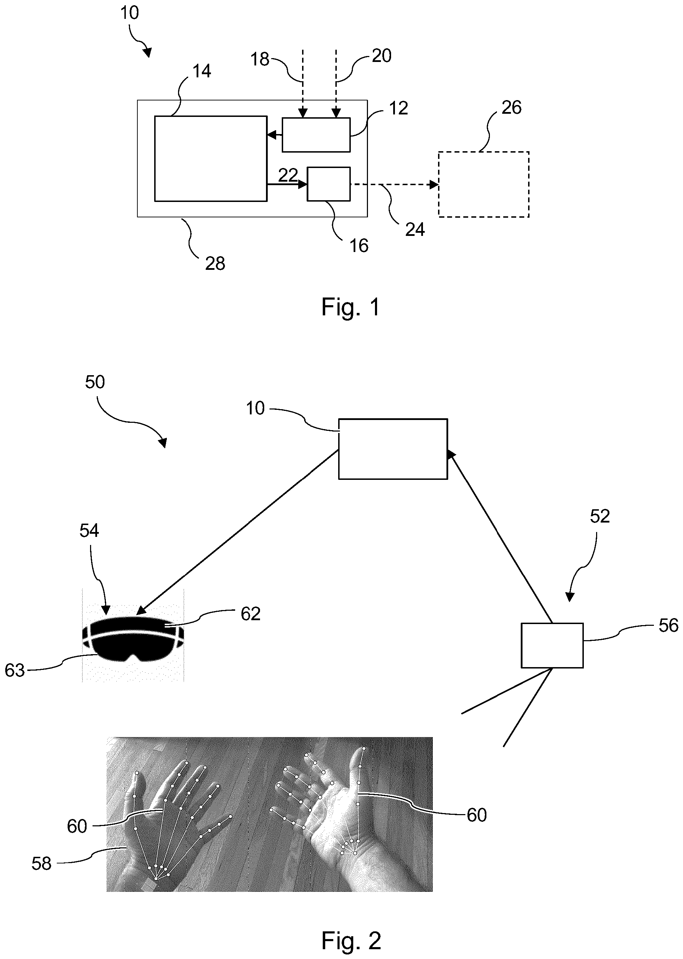

Fig. 1 schematically shows an example of a device 10 for interactive segmentation of 3D images. The device 10 comprises a data input 12, a data processor 14 and an output interface 16. The data input 12 is configured to provide 3D data 18 representing a region of interest of an object. The data input 12 is also configured to provide a 3D user input 20 relating to a 3D presentation. The data processor 14 is configured to render a 3D presentation of a segmentation, which segmentation is based on the 3D data, e.g. for 3D displaying the 3D presentation to a user. The data processor 14 is also configured to transform the 3D user input into 3D object data. The data processor 14 is further configured to adapt the 3D segmentation based on the transformed 3D object data. The data processor 14 is still further configured to render an updated 3D presentation data 22 of the adapted segmentation for a modified 3D display to a user. The output interface 16 is configured to provide the updated 3D presentation 22 data to a user.

As an further option, indicated with hashed lines, the updated 3D presentation 22 is provided via data connection 24 to a display 26.

In a further option, as indicated with a hashed frame 28, the data input 12, the data processor 14 and the output interface 16 are provided in an integrated manner, for example in a common housing. In an alternative option, the data input 12, the data processor 14 and the output interface 16 are provided in a separate manner, e.g. as separate components data connected with each other.

The data input 12 provides the 3D segmentation to the data processor 14 for the steps of: rendering a 3D presentation of the segmentation for 3D displaying to a user; providing a 3D user input relating to the 3D presentation; transforming the 3D user input into 3D object data; adapting the 3D segmentation based on the transformed 3D object data; and rendering updated 3D presentation data of the adapted segmentation for a modified 3D display to a user. The output interface 16 provides the updated 3D presentation data to a user.

It is noted that in one option, the 3D segmentation is provided pre-generated, i.e. already computed and available for further data processing. It is further noted that in another option, the 3D segmentation is generated by the data processor on the fly based on 3D data representing the region of interest of the object.

As an example, a suitable field of use is a mixed reality scenery in a catheterization laboratory as a lesion segmentation tool. Another field of use is a mixed reality scenery to delineate vessel cross-sections or abdominal aneurysms, as well as neuro-endovascular applications to segment and measure cerebral aneurysms.

In an ultrasound domain, a field of use is the analysis of images acquired with 3D transducers and the inclusion in on-cart or off-cart measurement and quantification software in a scenario where the workstation or cart hardware could be augmented with one or several virtual reality or augmented reality satellite devices.

With the rapid development of artificial intelligence and deep learning for image segmentation, the technique is also provided to improve usability of 3D annotation tools, bringing more confidence and efficiency in the 3D annotation process that is key to the success for any machine learning approach.

The user inputs are points in 3D space that are placed with a 3D (three-dimensional) interaction procedure.

By aligning the 3D spaces for the 3D user input and the 3D interaction presentation, a direct interaction is achieved. Further, the result of the segmentation is also three-dimensional, for instance a triangular surface mesh defined in the same space as the interaction space.

As an advantage, the user is provided with a facilitated workflow. As an example, the user does not have to choose a specific plane to interact with, but is rather working directly in 3D space. Further, the user is provided with visualizing segmentation and volume dataset in 3D space, without having to scroll through slices or the like. Natural interactions and stereoscopic visualization are combined to reduce the cognitive load. An increase in the degrees of freedom of inputs that is passed to the segmentation algorithm, is leveraging complex gestures and articulated hand tracking. As an example, for a sphere input, the user can change both radius and center in a single interaction by using 2 fingers located in 3D space.

As user input, finger positions are obtained by articulated hand tracking and the result is shown as stereoscopic volume-rendered blending of the segmentation result and the input volume, for example, in a virtual reality or mixed reality headset.

The data input 12 can be provided comprising an image data input and a user interaction data input. The image data input is configured to provide the 3D segmentation representing a region of interest of an object. The user interaction data input is configured to provide the 3D user input relating to the 3D presentation.

The data input 12 can also be referred to as data input module. The data processor can also be referred to as data processing module. The output interface can also be referred to as output interface module.

The data input 12 can also be referred to as data supply, as image supply, as image data supply, as segmentation input, as input unit or simply as input. In an example, the data input is data-connectable to an imaging source arrangement like a CT imaging system or MR imaging system providing the 3D image data of the subject which is used for the segmentation. In an example, the image data input is data-connectable to a data storage having stored the 3D segmentation image data.

The data processor 14 can also be referred to as data processing arrangement, as processor unit or as processor. In an example, the data processor is data-connected to the image data input and the output interface. In an example, the data processor is provided as mapping or registration engine that matches the current images with stored image data to identify an optimal path or trajectory for emergency ventricular drain placement. For example, the data processor is configured to generate a three-dimensional optical shape of the head. For example, the data processor is configured to provide a plurality of three-dimensional (3D) datasets of heads with different head shapes.

The output interface 16 can also be referred to as output or output unit. In an example, the output interface is data-connectable to a display arrangement or display device. In another example, the output is data-connected to a display.

The device for interactive segmentation of 3D images 10 can also be referred to as device for 3D-interactive segmentation of 3D images.

As a result, the user interaction provides interactive segmentation system to delineate and measure structures in 3D volumetric acquisitions.

In an example, the updated 3D presentation provides a blending of the volume and additional image data, such as a mesh defined by the user.

In an example, the data input 12 is configured to provide the 3D data as a 3D segmentation of the region of interest of the object.

In another example, the data input 12 is configured to provide the 3D data representing the region of interest of the object and the data processor is configured to generate the 3D segmentation of the region of interest of the object based on the 3D data.

Fig. 2 schematically shows a system 50 for interactive segmentation of 3D images. The system 50 comprises an example of the device 10 for interactive segmentation of 3D images according to one of the preceding examples. The system 50 further comprises at least one 3D interactive user input device 52 and at least one 3D presentation device 54. The 3D interactive user input device 52 is configured to provide the 3D user input. The 3D presentation device 54 is configured to provide the updated 3D presentation data as the modified 3D display to the user.

In an example, shown as an option in Fig. 2, the 3D interactive user input device 52 is provided as a hand tracking device 56 tracking at least one hand 58 of a user by identifying predetermined identification points and structure 60 of the user's hands, such as fingertips, finger and hand joints and the like.

In an example, shown as another option in Fig. 2, the 3D presentation device 54 is provided as a headset comprising a display such as a projection within the user's field of view for providing an augmented reality display to the user showing the view on reality overlaid by further information such as the modified 3D display. As an example, the 3D presentation device 54 is a headset that may also comprise other features like sound generation devices like earphones or speakers.

In an option, the 3D interactive user input device 52 is provided in an integrated manner with the 3D presentation device 54, for example with optical tracking sensors, e.g. cameras, integrated in the headset.

In an option, the 3D interactive user input device 52 and the 3D presentation device 54 are provided as a head-mounted gear 63 configured to be worn by the user.

In an option, for providing the 3D user input, the 3D presentation of the segmentation is configured to be registered with a 3D user input device.

The head-mounted gear is configured to provide a mixed reality to the user. In an example, the head-mounted gear is thus provided as a mixed reality headset, for example as an augmented reality headset.

In an option, the device 10 for interactive segmentation of 3D images is provided remotely from the head-mounted gear 63.

With a segmentation algorithm that is fast enough to provide a result in real-time, the immediate stereoscopic visual feedback of the impact of 3D user inputs and image features on the segmentation result creates an intuitive segmentation experience with natural hand-eye coordination for the user. The presentation, the input and the updated rendering are provided in the same 3D frame of reference. In an example, a (real) 3D image is provided in a (real) 3D presentation with a (real) 3D user interaction.

In another example, shown as an additional or alternative option to the hand tracking device 56, the 3D interactive user input device 52 comprises an eye gaze tracking device 62.

In a further option, the 3D presentation device 54 is a stereoscopic display. In another example, the 3D presentation device 54 comprises a display or projection surface.

It is thus provided to feed the segmentation with an impact from the user's hands. As a result, an intuitive feeling is provided.

In an example, the hand tracking device tracks hands and fingers of the user. In another example, the hand tracking device tracks gestures of the user.

In an example, the gaze tracking device tracks the user's viewing direction and focused points in relation to the provided 3D presentation.

In an example, the 3D input device is able to trigger events associated with points in 3D space that are co-registered with the stereoscopic display. This can be the hand tracking, eye tracking or physical controller modules of an AR/VR headset.

In an example, for the 3D user input, a tracking arrangement is provided configured to track a user's finger spatial positions in relation to the 3D presentation of the segmentation; and the input controller is configured to transfer the tracked finger positions into parameterized user input.

In an example, for the 3D user input, a tracking arrangement is provided configured to track a user's gaze in relation to the 3D presentation of the segmentation; and the data processor is configured to transfer the tracked gaze data into parameterized user input.

In an example, the stereoscopic display is capable of simultaneously visualizing the input volume and the segmentation result, with real-time display updates according to continuous changes of user inputs.

The term parameterized user input refers to a user input in form of parameters, e.g. values, characterizing the respective interaction.

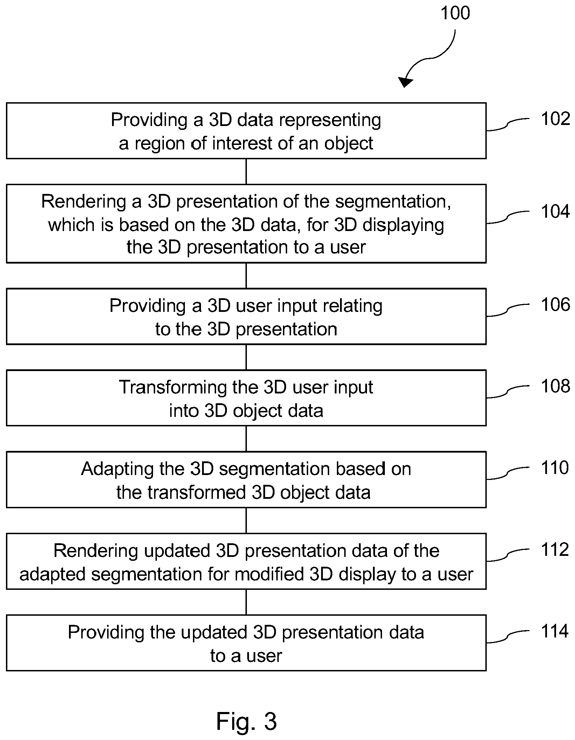

Fig. 3 shows steps of an example of a method 100 for interactive segmentation of 3D images. The method 100 comprises the following steps: In a first step 102, 3D data is provided representing a region of interest of an object. In a second step 104, a 3D presentation of the segmentation is rendered, which segmentation is based on the 3D data, for 3D displaying the 3D presentation to a user. In a third step 106, a 3D user input is provided relating to the 3D presentation. In a fourth step 108, the 3D user input is transformed into 3D object data. In a fifth step 110, the 3D segmentation is adapted based on the transformed 3D object data. In a sixth step 112, updated 3D presentation data of the adapted segmentation is rendered for a modified 3D display to a user. In a seventh step 114, the updated 3D presentation data is provided to a user.

In an example of the method, for providing the 3D segmentation, it is provided the steps of: providing the 3D data representing the region of interest of the object; and generating the segmentation of the region of interest based on the 3D data.

In an example of the method, for providing the 3D segmentation, the 3D data representing the region of interest of the object is provided as the 3D segmentation.

The adapting of the 3D segmentation based on the transformed 3D object data can also be referred to as adapting the 3D segmentation based on the user input.

The 3D displaying to a user of the rendered 3D presentation is provided as stereoscopic displaying.

In an example of the method, for providing the 3D user input, the 3D presentation of the segmentation is registered with a 3D user input device.

In an example of the method, for the 3D user input, a tracking of a user's finger spatial positions in relation to the 3D presentation of the segmentation is provided; and the finger positions are transferred into parameterized user input.

Hand or finger tracking provides a real-time 3D user input to segmentation algorithms with more degrees of freedom than conventional 3D segmentation user interfaces.

In an example, articulated hand tracking is provided, with real-time estimation of the location of finger joints and tips in 3D space. As an example, this is provided by a virtual reality (VR) or augmented reality (AR) headset. Hand tracking is advantageously used to provide real-time 3D inputs to 3D segmentation algorithms with more degrees of freedom than, for example, mouse or touch input devices.

For the presentation of the segmentation, both the initial and the adapted segmentation, stereoscopic capabilities of virtual reality or augmented reality devices or mixed reality devices, e.g. as headsets combine visualization of the segmentation result, such as a 3D mesh, and the input volume, i.e. volume-rendered stack of image slices.

In an example, for the 3D user input, a tracking of a user's gaze in relation to the 3D presentation of the segmentation is provided. The tracked gaze data is transferred into parameterized user input.

In an example of the method 100, the user input is provided as at least one of the group of delineation, measurement structure, annotation and labelling.

In an option of the device 10, for the 3D segmentation, it is provided that the data input is 12 configured to provide the 3D data representing the region of interest of the object. It is further provided that the data processor 14 is configured to generate the segmentation of the region of interest based on the 3D data.

In another option of the device 10, for the 3D segmentation, it is provided that the data input 12 is configured to provide the 3D data representing the region of interest of the object as the 3D segmentation. It is further provided that the data input 12 is configured to provide a user interactive segmentation input.

In an example, the data input 12 is configured to provide the 3D data as 3D volume data representing the region of interest of the object and the data processor 14 is configured to generate the segmentation of the region of interest based on the 3D volume data.

Hence, voxels are taken as an input together with information and/or input by the user. One approach for the segmentation is the front propagation approach, as an example.

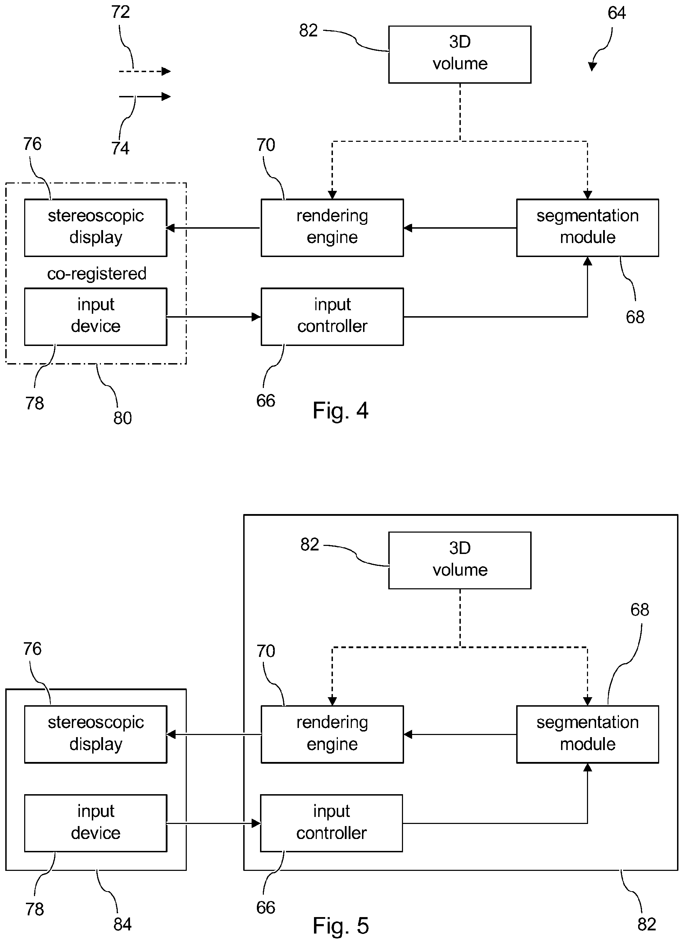

Fig. 4 shows a functional setup of another example of a system for interactive segmentation of 3D images. Fig. 4 shows a further example of the device 10, where the data processor 14 of the device for interactive segmentation is provided as a data processing arrangement 64 comprising an input controller 66, segmentor 68, and a rendering engine 70. The segmentor 68 can also be referred to as segmentation module. The input controller 66 can also be referred to as input module. The rendering engine 70 can also be referred to as render module. The input controller 66 is configured to transform the 3D user input into 3D object data. The segmentor 68 is configured to generate the segmentation of the region of interest based on the 3D volume. The rendering engine 70 is configured to render the 3D presentation of the segmentation for 3D displaying to the user, and to render the updated 3D presentation data of the adapted segmentation for the modified 3D display to the user.

In an example, during an initialization phase, the 3D volume data is passed to both the segmentor and the rendering engine. If the data is static, this step only needs to be done once. For dynamic datasets such as temporal sequences, it will be repeated each time the 3D volume source changes. Then, in a continuous loop, e.g. at 60 frames per second, for each frame it is provided: The 3D interactive user input device captures hand and/or eye position information, passes it to an input controller that interprets these events in a form that can be consumed by the segmentation module. The segmentation module calculates its output and communicates it to the rendering engine unit.

In another example, the segmentor 68 receives as inputs a 3D volume and additional user input that impacts the segmentation result by changing the initial conditions of the underlying algorithm or by setting additional constraints to modify the result.

In an example, the rendering engine 70 is capable of simultaneously rendering the input volume and the segmentation result, with real-time updates according to continuous changes of user inputs.

In an example, the input controller 66 interprets all input events from the input device and turns them into simple primitives (points, curves, spheres) that can be received by the segmentation module.

The input controller 66 thus provides the rules for defining what is meant by the respective different user input forms.

In an example, the rendering engine 70 unit blends the volume and the result of the segmentation together and sends the combined image to the stereoscopic display for the user.

The rendering engine 70 is configured to generate an adapted rendering of the modified or annotated segmentation overlaid on a volume-rendered 3D dataset, e.g. a semitransparent volume-rendered 3D dataset. In one option, the adapted rendering is provided as an opaque segmentation rendering. In another option, the adapted rendering is provided as a wireframe segmentation rendering or mesh segmentation rendering.

The rendering engine 70 provides the so-called look-and-feel for the user.

In an example, the input controller 66 is configured to provide the user with an option of creating a mesh based on the user's interaction by the user's fingers and/or eye gaze. The input controller 66 is also configured to provide the user with a further option of moving the mesh along the segmentation.

In a further example, the input controller 66 is configured to provide the 3D user input as at least one of the group of: boundary markings, delimitation parameters, selections, identification of type of measured parameter and measurement points, symbols, marks, comments, classifications, categories and labels. The output interface 16 is configured to provide the 3D updated 3D presentation comprising at least one of the group of: delineation, measurement structure, annotation and labelling.

The 3D updated 3D presentation is provided as a specialized output configuration of the data processor 14.

In an example, annotation is provided to provide a plurality of annotated segmentations that can serve as a basis for an artificial intelligence algorithm. The annotations serve as model segmentation to train the algorithm.

When the user is satisfied, a termination event is triggered to end the continuous loop and to fix the 3D segmentation to its current state, for instance using a voice command, e.g. "place" or "fixed".

The user providing additional information to the segmentation thus provides a modified or upgraded segmentation. Due to the respective user input, the segmentations improve the user's trust in the image data.

In Fig. 4 (and also in Fig. 5), broken lines 72 indicate initialization, and through lines 74 indicate a continuous update as an exemplary workflow with respective data flow. As an option, Fig. 4 shows a stereoscopic display 76 and an input device 78 being co-registered, as indicated with hashed-type line 80. Further, as an input, a 3D volume 82 is shown in Fig. 4.

In an example, the 3D volume 82 is provided to the segmentation module 68 and the rendering engine 70. The segmentation module 68 provides a segmentation result to the rendering engine 70. The rendering engine 70 provides a rendered volume plus a segmentation to the stereoscopic display 76. The 3D input device 76 provides at least one of the group of finger positions and eye gaze tracking to the input controller 66. The input controller 66 provides parameterized user input to the segmentation module 68. In a loop-like manner, the segmentation module 68 then provides updated segmentation results to the rendering engine 70, and so forth.

Fig. 5 shows a functional setup of a further example of a system for interactive segmentation of 3D images. Fig. 5 shows another example in which the 3D volume 82, the segmentation module 68, the rendering engine 70 and the input controller 66 are provided in form of a remote computer 82; and the stereoscopic display 76 and the input device 78 are provided as mixed reality headset 84.

In the example of Fig. 5, the 3D volume 82 is provided to the segmentation module 68 and the rendering engine 70. The segmentation module 68 provides a segmentation result, e.g. a mesh, to the rendering engine 70. The rendering engine 70 provides a rendered volume plus a segmentation to the stereoscopic display 76. The 3D input device 76 provides finger positions to the input controller 66. The input controller 66 provides a sphere, e.g. with center and diameter, to the segmentation module 68. In a loop-like manner, the segmentation module 68 then provides updated segmentation results to the rendering engine 70, and so forth.

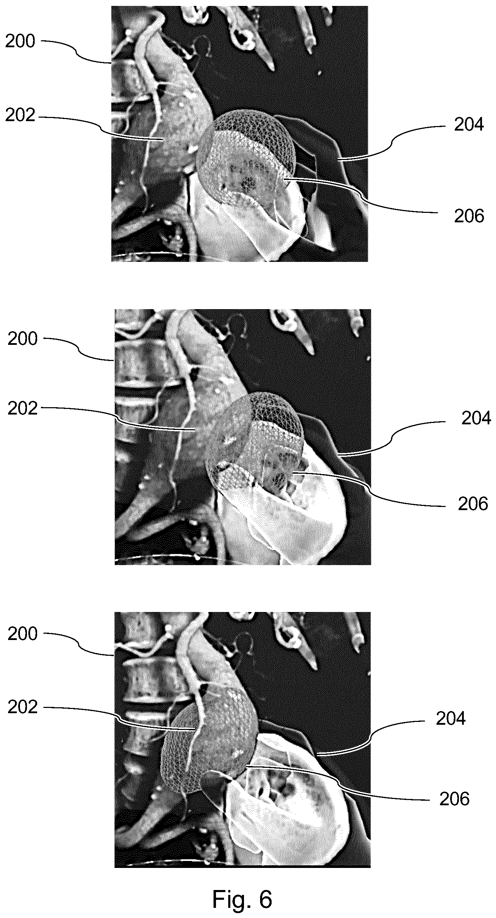

Fig. 6 shows an example of a user 3D interaction in 3D image. Fig. 6 shows an example of user interaction with a segmented volume rendering in 3D. A rendered 3D segmentation 200 is shown with an anatomical structure comprising e.g. an aneurysm 202. The rendered 3D segmentation 200 is provided overlaid to a view of the user's hand 204, i.e. overlaid to reality, or overlaid to a representation of the user's hand. A mesh-structure 206 is indicated representing a 3D volume.

In the upper part of Fig. 6, the mesh-structure 206 is having the form of a sphere and the user virtually touches the sphere with his/her hands for further interaction.

In the middle part of Fig. 6, the mesh-structure 206 is having the form of a slightly deformed sphere, caused by user interaction moving the mesh-structure 206 towards the aneurysm 202.

In the lower part of Fig. 6, the mesh-structure 206 is having the form of the aneurysm 202, as it has been moved to this location in the 3D volume space of the space of 3D segmentation 200 space with the user's interaction in 3D.

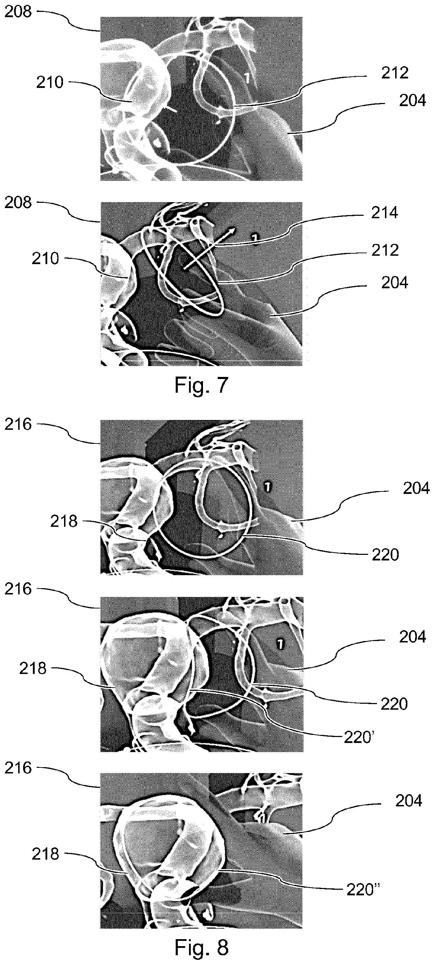

Fig. 7 shows another example of a user 3D interaction. Fig. 7 shows a further rendered 3D segmentation 208 with an anatomical structure 210. The rendered 3D segmentation 208 is provided overlaid to a view of the user's hand 204, i.e. overlaid to reality, or overlaid to a representation of the user's hand. A circle 212 is shown as an example for a pre-determined geometric form. An arrow 214 indicates a normal of a plane of the circle, i.e. a perpendicular line to the plane defined by the circle. The pre-determined geometric form can be manipulated in 3D, e.g. moved in its orientation and location. The user can adapt the circle to a structural part of the anatomy provided in the 3D segmentation 208 for example in order to illustrate the cross section or to measure a size like the diameter.

In the upper part of Fig. 7, the circle 212 is about parallel to the image plane of the drawing.

In the lower part of Fig. 7, the circle 212 is about perpendicular to the image plane of the drawing.

Fig. 8 shows a further example of a user 3D interaction. Fig. 8 shows a further rendered 3D segmentation 216 with an anatomical structure 218. The rendered 3D segmentation 216 is provided overlaid to a view of the user's hand 204, i.e. overlaid to reality, or overlaid to a representation of the user's hand. An adaptable geometric form 220 is shown.

In the upper part of Fig. 8, the adaptable geometric form 220 is having a circular shape as an initial or starting shape.

In the middle part of Fig. 8, the adaptable geometric form 220 is shown having a modified portion 220' due to user interaction and snapping to parts of the anatomical structure in 3D.

In the lower part of Fig. 8, the adaptable geometric form 220 is shown as modified form 220" due to further user interaction in 3D. The adapted form may thus be used for illustrating the chosen anatomical structure of interest.

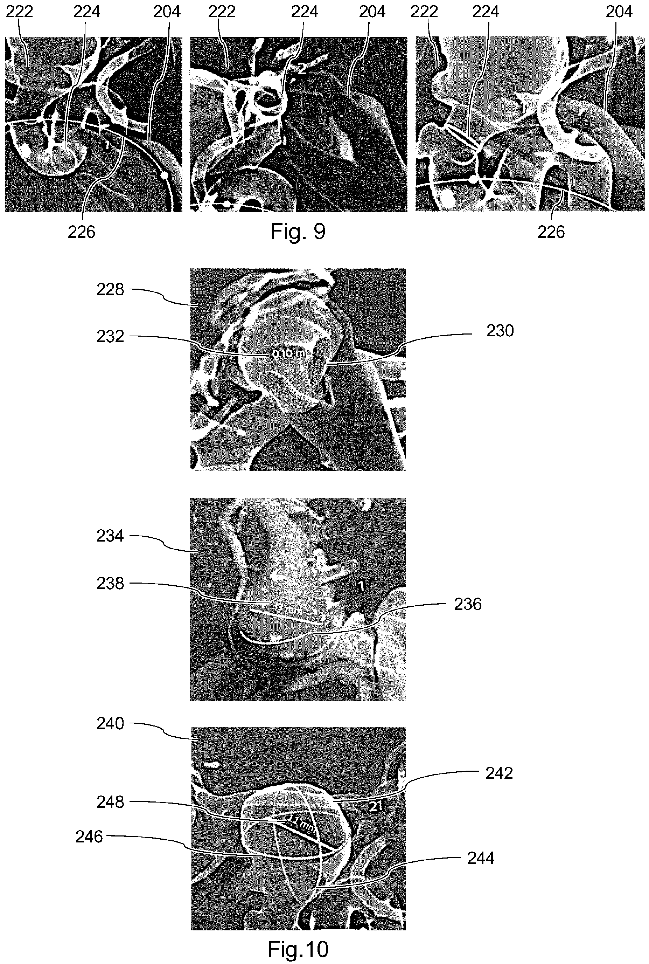

Fig. 9 shows a still further example of a user 3D interaction. Fig. 9 shows a still further rendered 3D segmentation 222 with an anatomical structure. The rendered 3D segmentation 222 is provided overlaid to a view of the user's hand 204, i.e. overlaid to reality, or overlaid to a representation of the user's hand. An adaptable geometric form 224 is shown in different geometric positions. The cross section is shown, for example, on a plane perpendicular to a longitudinal direction of the respective vessel section. The adaptable geometric form 224 is placed in the 3D segmentation based on user interaction in 3D.

In the left part of Fig. 9, the adaptable geometric form 224 is indicating a cross section of a particular vessel.

In the center part of Fig. 9, the adaptable geometric form 224 is indicating another cross section of a particular vessel.

In the right part of Fig. 9, the adaptable geometric form 224 is indicating a further cross section of a particular vessel.

Fig. 10 shows a further example of a user 3D interaction. Fig. 10 shows a further rendered 3D segmentation with an anatomical structure. An adaptable geometric form is shown in different options.

In the upper part of Fig. 10, a further rendered 3D segmentation 228 with an anatomical structure is shown, with a mesh-structure 230 indicates a possible anatomical structure of interest. An indication 232 of a content, i.e. a volume for the mesh-structure 230 is shown. The rendered 3D segmentation 228 is provided overlaid to a view of the user's hand 204, i.e. overlaid to reality, or overlaid to a representation of the user's hand.

In the middle part of Fig. 10, a still further rendered 3D segmentation 234 with an anatomical structure is shown, with a circular cross-section 236 of e.g. an aneurysm. An indication 238 of a diameter of that cross-section 236 is shown.

In the lower part of Fig. 10, another further rendered 3D segmentation 240 with an anatomical structure is shown. An amorph volume 242 indicates a selected part of the anatomical structure. A first cross-sectional contour 244 and a second cross-sectional contour 246 are indicated that are perpendicular to each other. An indication 248 of a diameter for the second cross-sectional contour 246 is shown.

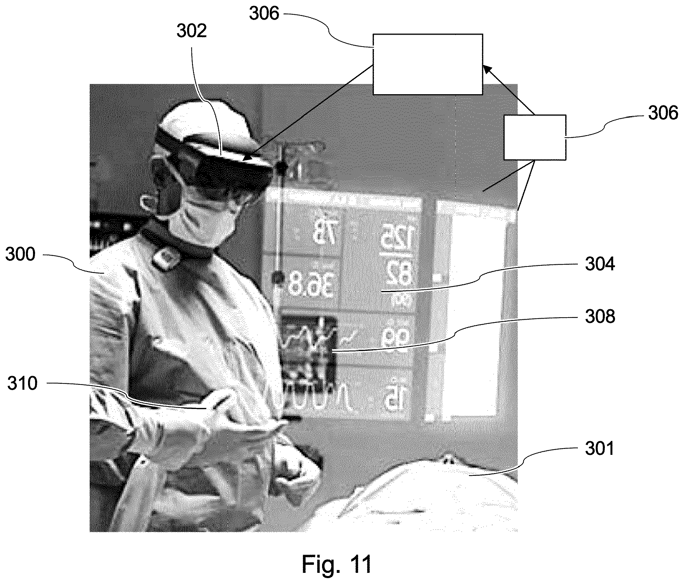

Fig. 11 shows a still further example of a user 3D interaction. A user 300, for example a surgeon or radiologist, is shown in an operation room, such as a cathlab (term used for catheterization laboratories). The user 300 is shown in the vicinity of a subject support with a subject 301 arranged in front of the user 300. The user 300 is wearing a headset 302 for augmented reality, thus providing an example for the 3D presentation device 54. The user 300 is provided with an overlay over the view on the physical scenery in front of him. The overlay as seen by the user is indicated by a projection 304 appearing in space in the figure. An example 306 of the device 10 for interactive segmentation of 3D images is schematically indicated in the upper right part. It is noted that the device 10 can be arranged anywhere in the room or even remote. The connecting lines indicate the necessary communication and can be provided wireless, or wire bound. Further, a hand-tracking device 306 is provided that tracks the user's hands. The hand-tracking device 306 thus provides an example of the 3D interactive user input device 52. On a part 308 of the displayed overlay, a graphical feedback of the user interaction in 3D space is provided. By moving and articulating a user's hand 310, i.e. in particular the fingers, the user is provided with a 3D interaction with the 3D image space provided via the 3D display, like stereoscopic view provided by the headset 302.

It is noted that the hand-tracking device 306, i.e. the 3D interactive user input device 52, can be provided integrated in the headset 302 or also separate in the room, e.g. above a subject support.

In another example, the data processor 14 is configured to provide interactive deformable 3D shape templates. The data processor 14 is configured to adapt the deformable shape templates based on at least one of the group: interactive 3D user input and optimized matching with the segmented 3D anatomical structure. The deformable 3D shape templates comprise models representing implantable devices. The data processor 14 is configured to adapt the models. The output interface 16 is configured to provide the 3D data of the models for manufacturing a subject-adapted implantable device.

In another option of interactive deformable shape templates, an addition of a shape constraint is provided, i.e. the ability to control that the result of the segmentation does not deviate too much from a known 3D shape. The input controller interprets input device events to calculate the pose of a shape template, or the pose of statistic shape model. For instance, the shape model can be a tube, or it can be a device CAD model (e.g. stent or other implantable devices). A statistical shape model is a specialized model for certain organs (a vertebra, a femur etc.). Segmentation algorithms with deformable shape templates or statistical shape models optimize parameters of the model (e.g. the eigen modes of a principal component analysis) via an iterative numerical scheme that depends on initial conditions, on an initial guess. Different initial conditions often lead to a different result, called a "local minimum". Optimizing the model of a vertebra for instance will depend on the initial position and is more likely to segment the closest one. Here, the user input derived from articulated hand tracking and provided to the segmentation algorithm will consist in the parameters defining this initial state.

In the case of a tubular template for instance, the finger positions can define the position, length orientation and diameter of the tube. The segmentation algorithm will either further locally adjust the same parameters by trying to optimize the fit of the template to the input volume dataset or optimize a larger set of parameters and degrees of freedom, for example by adding a non-rigid deformation component.

The previously placed volumes or circles could be used as other limits for the new deformable shapes, e.g. during an aneurysm embolization procedure, the aneurysm neck could be measured and marked using a circle and then an aneurysm volume would be defined as the area surrounded by the aneurysm boundaries and the circle surface. This could prevent penetration of the deformable volume into the parent vessel.

In another option of an input mode for initial segmentation seeds, segmentation seeds and initial shapes are placed using a combination of the eye gaze and voice command. A singular point, a multitude of points, an initial sphere with predefined diameter, or a sphere with user defined diameter (through voice command) could be placed around the center point defined by the intersection between the eye gaze direction and the 3D content. Segmentation algorithms like region growing or the propagation method could then be used to expand the point or spherical seeds to model boundaries.

Other input devices that can locate points in 3D space can be used instead of hand tracking, such as tracked virtual reality physical controllers.

For eye gaze with which a continuous update of the segmentation result in real-time might be disturbing to the user, the continuous loop can be replaced by a one-time segmentation call triggered by a voice command.

The choice of a particular deformable shape template can also be automatically selected by describing the target region that user is looking at through voice commands e.g., "segment this aneurysm", "segment this vessel". Since most of the aneurysms have a semi-spherical shape, the sphere would be a better choice of deformable models or seeds for them, whereas the tube for segmenting the vessel. The keywords such as aneurysm, vessel, organ names, etc. and their proper deformable shapes could be added to a lookup table and used by this system to improve the quality of the segmentation and reduce the number of manual adjustments.

In an example, the input controller 66 is configured to provide a pre-determined geometric form: The data processor 14 is configured to morph the geometric form to the segmentation.

In one option, not further shown, the form comprises a line, such that an interactive line is generated that can be manipulated in the 3D image space by 3D user interaction.

In another option, see also Fig. 7, the form comprises a circle, such that an interactive circle is generated that can be manipulated in the 3D image space by 3D user interaction.

In a further option, e.g. as shown in Fig. 6, the form comprises a sphere, such that an interactive sphere is generated that can be manipulated in the 3D image space by 3D user interaction.

It is thus provided a content adaptation of the user input, also referred to as the user entry.

For example, the interactive line is inserted and used for measurements in the 3D image space. The line can be adapted in its location, orientation and extension.

For example, the interactive circle is entered that is adapted to the segmentation providing a circle plane for a 2D image, but also a segmentation in a 2D image. The circle can be adapted in its location, orientation, i.e. plane, extension and outer contour. The circle can be morphed to a non-circular form, e.g. having an amorph outer shape adapted to a respective anatomical structure identified in the segmentation. The circle can be used for measurements and also for defining image planes for the generation of projections. The circle can be used for determining a cross-sectional profile of an anatomical structure of interest. For example, the cross-sections can be provided to segment vessels along an imaginary centerline.

For example, the interactive sphere is entered that is adapted to the segmentation providing a sphere within the 3D data. The interactive sphere can be adapted in its location, orientation, extension and outer contour. The sphere can be morphed to a non-spherical form, e.g. having an amorph outer shape adapted to a respective anatomical structure identified in the segmentation. The sphere, e.g. the adapted sphere, can be used for assisting in 3D measurements.

As options for the geometric entry, it is provided at least one of the group of lines, circles and spheres, thus providing ID, 2D and 3D respectively as user input, but spatially registered to the 3D volume.

In an example, adaptive interactive spheres are provided. This can be implemented with a mixed reality headset communicating wirelessly with a remote computer. To maximize frame rate and ensure a real-time response of the system, the most computationally demanding tasks, which are the rendering engine and 3D segmentation module, are offloaded to a remote computer with high graphics and computing resources.

The stereoscopic display and 3D input device are components of a headset. In a possible implementation, communication between the headset and the remote workstation is wireless, but the principle applies to any tethered or untethered virtual reality or mixed reality headsets that has similar display and sensor capabilities. The 3D input device may be provided as depth camera. Its software driver provides a location of all finger joints of both hands, in the form of 3D points that are co-registered with the content being displayed to the user via the additional head tracking cameras of the headset.

In an example, the input controller is a component running on a remote workstation that takes as input all hand joints and fingertips provided by the input device and calculates parameters of a virtual sphere that follows the hand(s). If only one hand is in the field of view, the sphere can be defined by the center, which is the middle of the segment between index and thumb, and/or by the diameter, which is the distance between index and thumb.

Other possible choices are to optimize the sphere parameters according to more than two fingers, including joints, or both hands, for instance with a least-square fitting of, e.g., a sphere to the tips of all extended fingers.

If both hands are present in the field of view, a possible choice defines the sphere center at the middle of the segment between the indexes of each hand, while the diameter is the distance between those two fingers.

With two hands, an alternative is for each hand to define its own sphere and all subsequent segmentation operations would happen in parallel and create two distinct objects.

The segmentation module is a component running on the remote workstation that transforms the input sphere into a virtual 3D object whose shape follows prominent volume features. In an example, the use of a 3D segmentation algorithm that considers user input from hand tracking is key, the "technical element" of combining segmentation, hand tracking and stereoscopic visualization: it creates the illusion for the user that the sphere is reacting to the content, modified by, and snapped to the structures being rendered in front of their eyes.

Segmentation can be achieved in a variety of ways, for instance with active surfaces, deformable models, statistical shape models, level sets, Markov random fields, graph cuts or neural networks. These segmentation techniques can include some form of regularization or smoothing to ensure the regularity of the surface, which is important to provide a sense of realistic, non-abrupt response to finger movements, ultimately resulting in an intuitive interaction. In an example, for various positions of the user hand, successive frames of the continuously updated segmentation result show a wireframe surface overlaid on the volume.

In an example, a fast-marching algorithm is used to propagate a front in 3D, in all directions, from a seed point located at the center of the sphere. If the speed of propagation is constant, the result is a perfect sphere. To morph the perfect sphere into a shape that automatically adapts to volume features, the front propagates at a speed that varies in space, depending on local volume features: It propagates at a relatively constant speed in regions that have similar intensity values as the seed point and will locally slowdown in regions where intensity values start to vary or if strong image gradients are encountered. The front will stop when the volume enclosed by the front surface is equal to the volume of the input sphere, so that the sphere diameter controlled by the user directly impacts the extent of the segmentation result.

In an example, not shown, color-coding of the arrival times of the front is provided where the front line follows image contours, hence the desired effect for adaptive interactive spheres. A crosshair may be provided to indicate a sphere center, the input sphere can be shown differently, plus the result of the segmentation, which is the front line for which enclosed volume is equivalent to the input sphere.

In an example, the input sphere is not displayed to the user, but only the result of the segmentation module is shown. If there are no structures in the vicinity of the sphere, it will remain untouched by the segmentation module because the propagation speed in the void will be constant, and the sphere shape will progressively evolve as user fingers approach the visible structures.

In an example, the segmentation result and propagation speed depend on rendering parameters such as the threshold value below which all voxels appear transparent, since they affect which structures are seen by the user.

The rendering engine realizes the fusion of the input 3D dataset, which can be a grid of voxels or a stack of images, with the segmentation result, which can be another volume or a mesh. If the result of the segmentation is another volume, each voxel can take binary values (0 or 1) that represent its membership to the object (e.g. 1) or the background (e.g. 0). In this case, a stereoscopic version of the volume rendering algorithm can be used to blend the two volume sources together. In an example, the segmentation module outputs a triangular mesh, shaded and rendered stereoscopically as an opaque, transparent or wireframe object on top of the 3D dataset. For a combined rendering of the volume and the segmented object, stereoscopic photo-realistic rendering with advanced lighting effects can further improve visualization of objects being segmented, manipulated, and blended.

In another option of adaptive interactive circles, a variation is provided in which the input controller creates a planar circle instead of a sphere and the segmentation module creates a planar curve instead of a surface mesh. Circle center and diameter are defined, but another degree of freedom is provided: The orientation of the plane. A third point, such as the thumb proximal joint or the index knuckle joint, defines the orientation of the plane, while the diameter remains the distance between index and thumb fingertips.

The segmentation module in this example can be an image segmentation algorithm working in two dimensions, but where the propagation is restricted to the plane defined by the input circle. The resulting curve is rendered with varying colors along the curve. For each point along the curve, color represents the distance to the closest point in the 3D volume, to draw the user's attention to the local goodness-of-fit of the result. The circle diameter and the orientation of the curve plane can also be optimized by, for instance, adapting to the local curvature to remain perpendicular to the targeted structure. For vessels, user input would then serve as a manual selection of the contour of a cross-section along an imaginary centerline.

In another option of assisted 3D measurements, the interactive segmentation workflow is complemented with measurements performed by analyzing the segmentation result, extending the main functionality to an assisted 3D measurement tool for augmented or virtual reality headsets. The user indicates the structure of interest with inputs like a sphere or two circles, but the segmentation module is now an intermediary step, a means to obtain measurements of the target structure automatically. From the mesh result above, or the curve result above, quantities such as volume, area, minimum and maximum diameters can be calculated and displayed to the user in real-time.

This can also be used to automatically measure a combined set of measurements like vessel diameters at two points separated by a fixed distance, aneurysm widths orthogonal to each other.

In a first option of an example, the data processor 14 is configured to provide a snapping function from outside a segmentation of the 3D volume. In a second option of the example, in addition or alternatively, the data processor 14 is configured to provide a snapping function from inside a segmentation of the 3D volume. For the snapping from inside, the user spatially creates a geometric form within the segmentation as provided in 3D, and the geometric form snaps to the segmentation, becoming larger or increased in size. For the snapping from outside, the user spatially creates a geometric form enclosing the segmentation as provided in 3D, and the geometric form snaps to the segmentation, becoming small or decreased in size. The snapping from within or from outside depends on the type of rendering provided. In an example, the snapping function is provided as snapping to a voxel volume. In another example, the snapping function is provided as snapping to a boundary.

In an example of the system shown in Fig. 2, the 3D interactive user input device 52 is configured to provide the 3D user input as segmentation constraints as correction instructions to modify the segmentation. The data processor 14 is configured to revise the 3D segmentation representing the region of interest of the object based on the segmentation constraints. The 3D presentation device 54 is configured to provide the revised 3D presentation data as the modified 3D display to the user.

In another option of a user input as inside/outside segmentation constraints, user input is interpreted not as an initial condition for a segmentation algorithm, but as additional constraints to "correct" an existing result. It can be used after an initial segmentation has been obtained with any of the previous embodiments. The constraint can for instance be expressed as points or curves that are inside, outside or on the boundary of the target structure.

For instance, a mathematical formulation of deformable shape templates can include such constraints. The non-linear optimization process under a set of constraints can be solved and could be used to push or pull the segmentation in certain directions, while continuing to optimize the deformation of the shape to find an optimal fit to the input volume dataset that satisfies the constraints. A possible set of constrained points could include that all hand joints should be outside of the result. This would allow users to "sculpt" the segmentation with any part of their hands, pushing the surface towards the inside.

In another option, a patient specific design of implantable devices like asymmetric stents/grafts etc. is provided. As an extension, the user places generic virtual models of the devices in the 3D image. e.g. a generic model of a fenestrated endovascular aortic repair graft is placed in an image of an aortic abdominal aneurysm. The user then can mark the locations of branching vessels like the renal vessels where the graft needs to be perforated. The result is saved and exported as a CAD model which can be used to manufacture the patient specific graft.

Another example is an asymmetric stent where only the portion where the aneurysm needs to be occluded is covered with a fine mesh. In this case the user places a generic virtual model of stent in the feeding vessel to the aneurysm and marks the location of the neck of the aneurysm on the stent model. This is then exported as a CAD model to manufacture the patient specific asymmetric stent.

In another option, not shown in further detail, a voice triggered anatomical segmentation is provided. The 3D volume application can utilize a neural network to detect and identify anatomical structures. Segmentation of these structures can be triggered by voice command, for instance, the user could say 'volume measure aneurysm' and the aneurysm in the field of view will be automatically segmented.

The term "subject" may also be referred to as individual. The "subject" may further also be referred to as patient, although it is noted that this term does not indicate whether any illness or disease is actually present with the subject.

In an example, a computer program or program element for controlling an apparatus according to one of the examples above is provided, which program or program element, when being executed by a processing unit, is adapted to perform the method steps of one of the method examples above.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for interactive segmentation of 3D images, the device comprising:
- a data input (12);
- a data processor (14); and
- an output interface (16);
wherein the data input is configured to provide 3D data (18) representing a region of interest of an object; and to provide a 3D user input (20) relating to a 3D presentation;
wherein the data processor is configured to render a 3D presentation of a segmentation, which segmentation is based on the 3D data, for 3D displaying the 3D presentation to a user; to transform the 3D user input into 3D object data; to adapt the 3D segmentation based on the transformed 3D object data; and to render an updated 3D presentation data (22) of the adapted segmentation for a modified 3D display to a user; and
wherein the output interface is configured to provide the updated 3D presentation data to a user.

2. Device according to claim 1, wherein, for the 3D segmentation, it is provided:
i) the data input is configured to provide the 3D data representing the region of interest of the object; and the data processor is configured to generate the segmentation of the region of interest based on the 3D data; or
ii) the data input is configured to provide the 3D data representing the region of interest of the object as the 3D segmentation; and
wherein the data input is configured to provide a user interactive segmentation input.

3. Device according to one of the preceding claims, wherein the data processor of the device for interactive segmentation is provided as a data processing arrangement (64) comprising:
- an input controller (66) configured to transform the 3D user input into 3D object data; and/or
- a segmentor (68) configured to generate the segmentation of the region of interest based on the 3D volume; and/or
- a rendering engine (70) configured to render the 3D presentation of the segmentation for 3D displaying to the user, and to render the updated 3D presentation data of the adapted segmentation for the modified 3D display to the user.

4. Device according to claim 3, wherein the input controller is configured to provide the user with an option of creating a mesh based on the user's interaction by the user's fingers and/or eye gaze; and to provide the user with a further option of moving the mesh along the segmentation.

5. Device according to claim 3 or 4, wherein the input controller is configured to provide the 3D user input as at least one of the group of:
- boundary markings, delimitation parameters, selections;
- identification of type of measured parameter and measurement points;
- symbols, marks, comments, classifications, categories;
- labels; and
wherein the output interface is configured to provide the 3D updated 3D presentation comprising at least one of the group of:
- delineation;
- measurement structure;
- annotation; and
- labelling.

6. Device according to one of the preceding claims, wherein the data processor is configured to provide interactive deformable 3D shape templates; and
wherein the data processor is configured to adapt the deformable shape templates based on at least one of the group:
- interactive 3D user input, and
- optimized matching with the segmented 3D anatomical structure; and
wherein the deformable 3D shape templates comprise models representing implantable devices; wherein the data processor is configured to adapt the models; and the output interface is configured to provide the 3D data of the models for manufacturing a subject-adapted implantable device.

7. Device according to one of the preceding claims, wherein the input controller is configured to provide a pre-determined geometric form; and the data processor is configured to morph the geometric form to the segmentation; and
wherein the form comprises at least one of the group of:
- a line, such that an interactive line is generated that can be manipulated in the 3D image space by 3D user interaction;
- a circle, such that an interactive circle is generated that can be manipulated in the 3D image space by 3D user interaction; and
- a sphere, such that an interactive sphere is generated that can be manipulated in the 3D image space by 3D user interaction.

8. Device according to one of the preceding claims, wherein the data processor is configured to provide a snapping function for at least one of the group of:
- from outside a segmentation of the 3D volume; or
- from inside a segmentation of the 3D volume.

9. A system (50) for interactive segmentation of 3D images, the system comprising:
- a device (10) for interactive segmentation of 3D images according to one of the preceding claims;
- at least one 3D interactive user input device (52); and
- at least one 3D presentation device (54);
wherein the 3D interactive user input device is configured to provide the 3D user input; and
wherein the 3D presentation device is configured to provide the updated 3D presentation data as the modified 3D display to the user.

10. System according to claim 9, wherein the 3D interactive user input device comprises at least one of the group of:
- a hand tracking device (56); and
- a gaze tracking device (62); and
wherein the 3D presentation device is a stereoscopic display.

11. System according to claim 9 or 10, wherein the 3D interactive user input device and the 3D presentation device are provided as a head-mounted gear (110) configured to be worn by the user; and/or
wherein for providing the 3D user input, the 3D presentation of the segmentation is configured to be registered with a 3D user input device; and
wherein the device for interactive segmentation of 3D images is provided remotely from the head-mounted gear.

12. System according to one of the claims 9 to 11, wherein the 3D interactive user input device is configured to provide the 3D user input as segmentation constraints as correction instructions to modify the segmentation;
wherein the data processor is configured to revise the 3D segmentation representing the region of interest of the object based on the segmentation constraints; and
wherein the 3D presentation device is configured to provide the revised 3D presentation data as the modified 3D display to the user.

13. A method (100) for interactive segmentation of 3D images, the method comprising the following steps:
- providing (102) 3D data representing a region of interest of an object;
- rendering (104) a 3D presentation of the segmentation, which segmentation is based on the 3D data, for 3D displaying the 3D presentation to a user;
- providing (106) a 3D user input relating to the 3D presentation;
- transforming (108) the 3D user input into 3D object data;
- adapting (110) the 3D segmentation based on the transformed 3D object data;
- rendering (112) updated 3D presentation data of the adapted segmentation for a modified 3D display to a user; and
- providing (114) the updated 3D presentation data to a user.

14. A computer program enabling a processor to carry out the method of claim 13.

15. A computer readable medium having stored the program element of claim 14.
